Europäisches Patentamt

European Patent Office

Office européen des brevets

(11) Numéro de publication : **0 147 334**
**B1**

(12) **FASCICULE DE BREVET EUROPÉEN**

(45) Date de publication du fascicule du brevet :
15.06.88

(21) Numéro de dépôt : 84402735.9

(22) Date de dépôt : 27.12.84

(51) Int. Cl.⁴ : **A 61 B 6/00**

(54) Installation de radiologie à récepteur unique.

(30) Priorité : 03.01.84 FR 8400036

(43) Date de publication de la demande :
03.07.85 Bulletin 85/27

(45) Mention de la délivrance du brevet :
15.06.88 Bulletin 88/24

(84) Etats contractants désignés :
DE IT NL

(56) Documents cités :
FR-A- 2 158 649
GB-A- 797 297
US-A- 2 582 776

(73) Titulaire : THOMSON-CGR
13, square Max-Hymans
F-75015 Paris (FR)

(72) Inventeur : Lajus, Pierre Claude
THOMSON-CSF SCPI - 173, bld Haussmann
F-75379 Paris Cedex 08 (FR)

(74) Mandataire : Grynwald, Albert et al
THOMSON-CSF SCPI 19, avenue de Messine
F-75008 Paris (FR)

Jouve, 18, rue St-Denis, 75001 Paris, France

## Description

L'invention concerne une installation de radiologie à récepteur unique ; plus particulièrement, l'invention concerne une telle installation permettant notamment des observations radiologiques de face et de profil au moyen d'un seul récepteur d'image, en mettant en jeu des moyens mécaniques simples et bien adaptés pour être associés à une table d'examen basculante.

Du brevet US-A-2 582 776 est connu un dispositif radiologique comprenant un statif porte-source et porte-récepteur ainsi qu'une table porte-patient. Toutefois, la table est indépendante du statif qui est également équipé d'un plateau appui-patient.

Dans les installations de radiologie, on utilise de plus en plus les amplificateurs de luminance en tant que récepteur. Ces dispositifs présentent en effet l'avantage de pouvoir être lus par une caméra de télévision, ce qui permet, après numérisation du signal vidéo et traitement numérique d'images (soustraction logarithmique d'images, en particulier) d'obtenir des images d'un contraste et d'une netteté exceptionnels. Les amplificateurs de luminance sont encore relativement lourds et encombrants de sorte que leur incorporation dans une installation de radiologie pose souvent des problèmes. En outre, c'est un sous-ensemble coûteux de l'installation.

Parmi les possibilités d'utilisation qu'il est souhaitable d'offrir au médecin, on peut citer principalement la possibilité de réaliser des examens radiologiques de face ou de profil et aussi le fait de pouvoir disposer d'une table d'examen basculante. L'invention permet de répondre à ces exigences, de façon particulièrement simple et économique, du fait notamment qu'elle n'utilise qu'un seul récepteur.

Dans cet esprit, l'invention concerne principalement une installation de radiologie comportant une table d'examen avec une surface porte-patient, au-moins une source de rayons X et un unique récepteur d'image monté sur un support mobile, ledit support mobile comportant une colonne de guidage le long de laquelle se déplace un chariot portant un bras qui soutient ledit récepteur, ledit bras étant mobile transversalement par rapport à la surface porte-patient et par rapport à la colonne de guidage, caractérisé en ce que la colonne de guidage est liée mécaniquement à la table d'examen (11) et en ce que ledit récepteur est monté sur ledit bras par l'intermédiaire d'un arbre de rotation disposé parallèlement à la longueur de la table d'examen et blocable dans au moins deux positions prédéterminées permettant respectivement un examen radiologique de face et un examen radiologique de profil d'un patient placé sur la surface porte-patient.

Cet agencement permet le double examen, de face et de profil. Bien entendu, la structure qui vient d'être décrite ci-dessus peut être associée à une table d'examen basculante, connue en soi, le support mobile précité étant solidaire de ladite table basculante et pouvant se déplacer longitudinalement par rapport à elle. Il est à noter en outre, que l'installation ainsi définie présente un triple accès (l'un des côtés longitudinaux de la table et les deux extrémités de celle-ci) ce qui est particulièrement avantageux pour l'utilisateur.

L'invention sera mieux comprise et d'autres avantages de celle-ci apparaîtront mieux à la lumière de la description qui va suivre d'une installation de radiologie conforme à son principe, donnée uniquement à titre d'exemple et faite en référence aux dessins annexés dans lesquels :

— la figure 1 est une vue générale en élévation d'une installation conforme à l'invention ;

— la figure 2 est une vue suivant la flèche II de la figure 1, montrant les différents éléments en position pour un examen radiologique de face ;

— la figure 3 est une vue analogue à la figure 2, montrant les éléments en position pour un examen radiologique de profil.

En se référant aux dessins, l'installation se compose principalement d'une table d'examen 11 comportant une surface porte-patient 12, d'une première source de rayons X13 et d'un récepteur d'image 14 porté par un support mobile 15. La table d'examen 11 est avantageusement (mais non exclusivement) une table basculante connue en soi. Sur le mode de réalisation représenté, la surface porte-patient est un panneau rigide, mais on pourrait facilement prévoir une surface plus ou moins concave définissant une sorte de couchette. Le récepteur d'images peut être, par exemple, un amplificateur de luminance classique. Le support mobile 15 comporte un bras 16 mobile transversalement par rapport à la table 11 et, pour cela, assujetti à se déplacer dans des moyens de guidage définis dans un chariot 17. Ce dernier est lui-même assujetti à se déplacer le long d'une colonne de guidage 18 liée à la table et disposée sur un côté longitudinal de celle-ci, sensiblement perpendiculairement à la surface porte-patient. Un prolongement inférieur 19 de cette colonne coopère dans des glissières en queue d'aronde 20 (ou tout autre système équivalent) solidaires de la table et agencées longitudinalement. Ainsi, l'ensemble du support mobile 15 portant le récepteur 14 suit le mouvement de basculement de la table 11. Tous les différents mouvements relatifs entre le récepteur 14, le bras 16, le chariot 17, la colonne 18 et la table sont de préférence contrôlés par des moteurs électriques associés à des systèmes d'asservissement de position classiques, qui ne sont pas décrits car ils ne font pas partie de l'invention. On peut aussi prévoir des systèmes à déplacement manuel, équilibré.

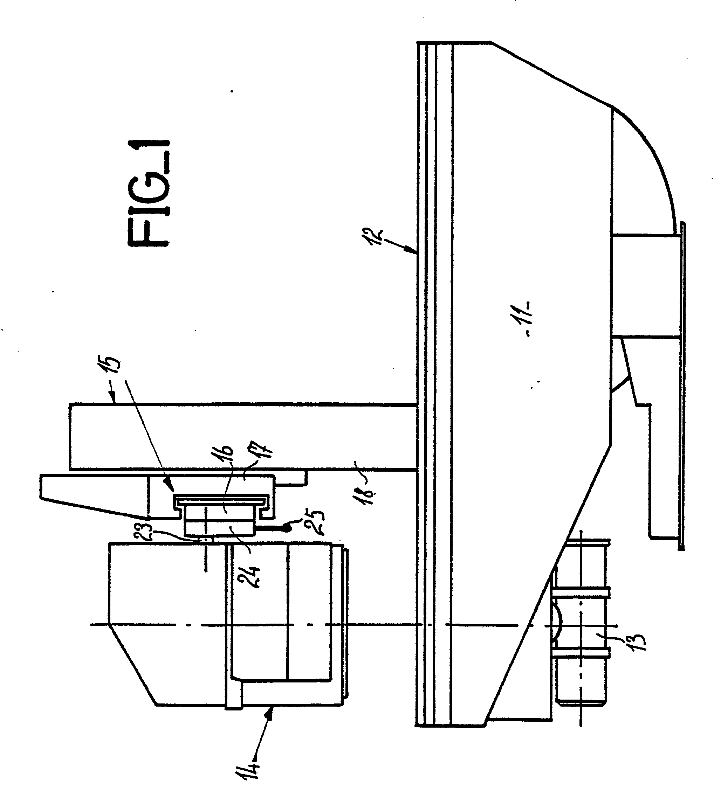

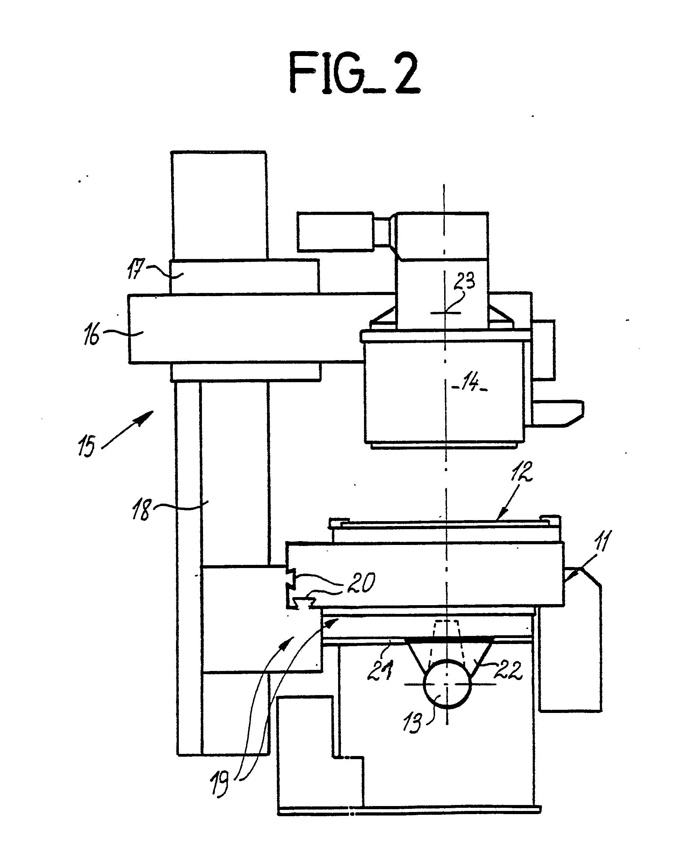

La source de rayons X13 intégrée à la table 11 est montée mobile longitudinalement dans la structure basculante de la table d'examen. Avantageusement, elle peut être montée sur le prolongement inférieur 19 de la colonne 18 pour suivre les déplacements longitudinaux du récepteur 14. Selon l'exemple, la source de rayons X13 est aussi mobile transversalement pour suivre les déplacements transversaux du récepteur d'image. Pour cela, elle est fixée à un support intermédiaire 22 mobile dans des glissières 21 dudit prolongement inférieur 19 de la colonne 18. De préférence, on prévoit un système d'asservissement de position entre les moyens de commande du bras 16 et ceux du support intermédiaire 22. La source 13 est orientée pour que l'axe du faisceau du rayons X soit sensiblement perpendiculaire à la surface porte-patient 12, c'est-à-dire continuellement centré par rapport au récepteur 14 (compte tenu des déplacements liés définis ci-dessus) lorsque celui-ci se trouve dans une position prédéterminée, par rapport au bras 16 (figures 1 et 2), c'est-à-dire face à la table.

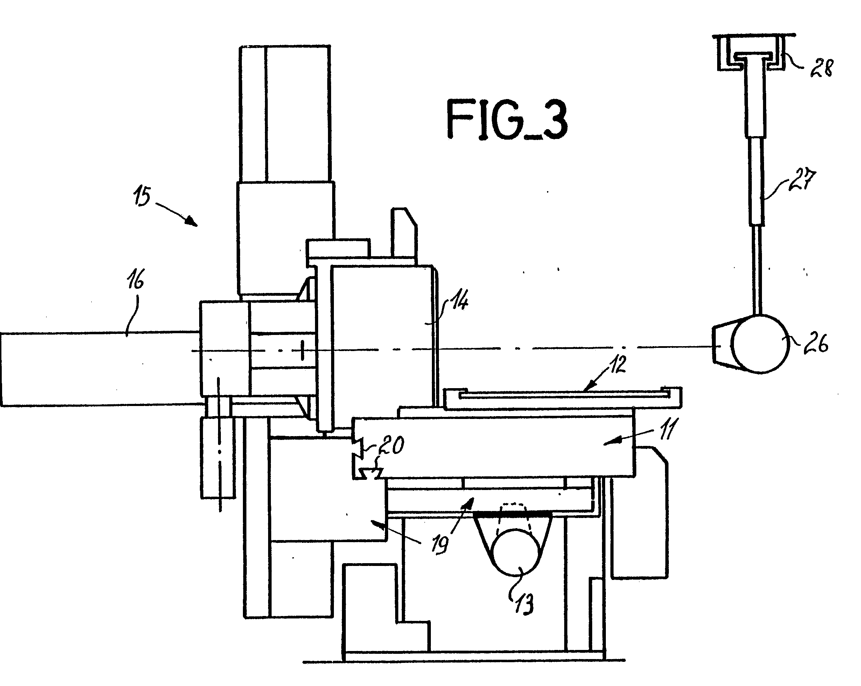

Selon une caractéristique importante de l'invention, le récepteur 14 est monté sur le bras 16 par l'intermédiaire d'un arbre de rotation 23 disposé parallèlement à la longueur de la table d'examen (11) et blocable dans au moins deux positions prédéterminées. L'une de ces positions est celle qui place le récepteur dans la position représentée aux figures 1 et 2. L'autre position, à 90° de la précédente, est celle qui place le récepteur dans la position représentée à la figure 3. L'arbre 23 tourillonne par exemple dans un dispositif à palier 24, fixé au bras 16 et muni de moyens de blocage commandés par un levier 25. Le dispositif 24 peut aussi comporter un moteur électrique commandant la rotation de l'arbre 23. La position de ce dernier par rapport au récepteur 14 est choisie pour approcher au mieux l'équilibrage indifférent du récepteur 14 par rapport à l'arbre 23, pour faciliter la manœuvre, notamment dans le cas où celle-ci n'est pas motorisée.

Ainsi, grâce à cet agencement, le récepteur 14 peut venir occuper une autre position (figure 3) latérale par rapport à la table, par le jeu d'un pivotement de 90° du récepteur par rapport au bras 16 accompagné d'un retrait transversal de celui-ci et d'un abaissement du chariot 17. Une seconde source de rayons X26 solidaire d'un support mobile indépendant de la table, peut alors être approchée de cette dernière pour permettre un examen radiologique de profil. On peut prévoir par exemple une suspension télescopique 27 déplaçable dans un rail 28 fixé au plafond de la salle de radiologie.

**Revendications**

1. Installation de radiologie comportant une table d'examen (11) avec une surface porte-patient (12), au-moins une source de rayons X et un unique récepteur d'image (14) monté sur un support mobile (15), ledit support mobile (15) comportant une colonne de guidage (18) le long de laquelle se déplace un chariot (17) portant un bras (16) qui soutient ledit récepteur (14), ledit bras (16) étant mobile transversalement par rapport à la surface porte-patient (12) et par rapport à la colonne de guidage (18), caractérisée en ce que la colonne de guidage (18) est mécaniquement liée à la table d'examen (11) et disposée sur un côté longitudinal de celle-ci, sensiblement perpendiculairement à la surface porte-patient (12) et en ce que ledit récepteur (14) est monté sur ledit bras par l'intermédiaire d'un arbre de rotation (23) disposé parallèlement à la longueur de la table d'examen (11) et blocable dans au moins deux positions prédéterminées permettant respectivement un examen radiologique de face et un examen radiologique de profil d'un patient placé sur la surface porte-patient (12).

2. Installation selon la revendication 1, caractérisée en ce que ladite table d'examen (11) est une table basculante.

3. Installation selon la revendication 2, caractérisée en ce qu'un prolongement inférieur (19) de ladite colonne coopère avec au moins une glissière (20) ou analogue solidaire de ladite table et agencée longitudinalement.

4. Installation de radiologie selon l'une des revendications 1 à 3, caractérisée en ce qu'elle comporte une source de rayons X (13) montée sur un support mobile (22) pour émettre un faisceau sensiblement perpendiculairement à ladite table, en regard dudit récepteur (14) lorsque celui-ci est dans une position qui correspond à l'une des positions prédéterminées précitées dudit arbre.

5. Installation selon l'une des revendications 1 à 4, caractérisée en ce qu'elle comporte une première source de rayons X (13) intégrée à la table sous la surface porte-patient de celle-ci et orientée pour que l'axe du faisceau soit sensiblement perpendiculaire à ladite surface porte-patient et une seconde source de rayons X (26) solidaire d'un autre support mobile (27, 28) indépendant de ladite table.

6. Installation selon l'une des revendications 2 à 4 et la revendication 5, caractérisée en ce que ladite première source de rayons X (13) est montée mobile longitudinalement dans la structure basculante de ladite table d'examen (11).

7. Installation selon la revendication 6, caractérisée en ce que ladite première source (13) est montée sur ledit prolongement inférieur (19) de ladite colonne et se déplace avec elle.

8. Installation selon la revendication 6, caractérisée en ce que ladite première source (13) est fixée à un support intermédiaire (22) mobile dans des glissières (21) transversales dudit prolongement inférieur (19).

**Claims**

1. An X-ray installation comprising an examination table (11) having a patients surface (12), an X-ray source and an image receiver (14) mounted on a mobile support (15), the latter comprising a guide pillar (15) with a carriage (17) sliding along and equipped with an arm (16) supporting the receiver (14), the arm being adapted to move transversely in relation to the patients surface (12) and to the guide pillar (18), characterized in that the guide pillar is mechanically linked up with the examination table (11) and disposed alongside of it and substantially perpendicular to the patients surface (12), and that the receiver (14) is mounted on said arm by means of a rotation axis (24) disposed parallelly to the long side of the examination table (11) and adapted to become blocked in at least two predetermined positions permitting a radiological examination respectively from the front side and in profile of a patient placed upon the patients surface (12).

2. An installation according to claim 1, characterized in that the examination table (11) is a tilting table.

3. Installation according to claim 2, characterized in that an lower extension (19) of the guide pillar cooperates with at least one slideway (20) or analog solidly fixed to the table and actuated longitudinally.

4. An X-ray installation according to one of claims 1 to 3, characterized in that it comprises an X-ray source (13) mounted on a mobile support (22) for emitting a radiation beam substantially perpendicular to said table, facing the receiver (14), when the latter is in a position which corresponds to one of the above mentioned predetermined positions of said rotation axis.

5. An installation according to one of claims 1 to 4, characterized in that it comprises a first X-ray source (13) connected to the table below the patients surface and oriented in such a way that the radiation beam axis extends substantially perpendicular to the patients surface, and that it comprises a second X-ray source (26) fixed to another mobile support (27, 28) independent from the table.

6. An installation according to one of the claims 2 to 4 and claim 5, characterized in that the first X-ray source (13) is mounted within the tilting structure of the examination table (11) and adapted to move longitudinally.

7. An installation according to claim 6, characterized in that the first source (13) is mounted in the lower extension (19) of the guide pillar and adapted to move with it.

8. An installation according to claim 6, characterized in that the first source (13) is fixed to a mobile intermediate support (22) engaging the transversal slideway (21) of the lower extension (19).

**Patentansprüche**

1. Röntgenanlage, bestehend aus einem Untersuchungstisch (11) mit Patientenfläche (12), mindestens einer Röntgenstrahlquelle und einem auf einem beweglichen Träger (15) montierten Bildempfänger (14), wobei der bewegliche Träger (15) eine Führungssäule (18) aufweist, an der entlang sich ein Schlitten (17) mit einem Arm (16) bewegt, welcher den genannten Empfänger (14) haltert, wobei der Arm quer zur Patientenfläche (12) und zur Führungssäule (18) beweglich ist, dadurch gekennzeichnet, daß die Führungssäule mechanisch mit dem Untersuchungstisch (11) verbunden und an einer Längsseite desselben, im wesentlichen senkrecht zur Patientenfläche (12) angeordnet ist, und daß der Empfänger (14) am Arm über eine Drehachse (23) befestigt ist, die parallel zur Längsrichtung des Untersuchungstisches (11) angeordnet und in mindestens zwei vorbestimmten Positionen blockierbar ist, sodaß eine Röntgenuntersuchung eines auf der Patientenfläche plazierten Patienten frontal und im Profil möglich ist.

2. Anlage nach Anspruch 1, dadurch gekennzeichnet, daß der Untersuchungstisch (11) ein Kipptisch ist.

3. Anlage nach Anspruch 2, dadurch gekennzeichnet, daß eine untere Verlängerung (19) der Säule mit mindestens einer Gleitschienenbahn (20) oder ähnlichem zusammenwirkt, die mit dem Tisch fest verbunden und in Längsrichtung angeordnet ist.

4. Röntgenanlage nach einem der Ansprüche 1 bis 3, dadurch gekennzeichnet, daß sie eine an einem beweglichen Träger (22) montierte Röntgenquelle (13) aufweist, die einen im wesentlichen senkrecht zum Tisch gerichteten Strahl aussendet, dem Empfänger (14) gegenüberstehend, wenn dieser sich in einer Stellung befindet, die einer der genannten vorbestimmten Positionen der Drehachse entspricht.

5. Anlage nach einem der Ansprüche 1 bis 4, dadurch gekennzeichnet, daß sie eine erste Röntgenstrahlquelle (13), die unter der Patientenfläche in den Tisch einbezogen und so orientiert ist, daß die Strahlenachse im wesentlichen senkrecht zur Patientenfläche verläuft und daß sie eine zweite Röntgenstrahlquelle (26) aufweist, die mit einem anderen beweglichen vom Tisch unabhängigen Träger (27, 28) fest verbunden ist.

6. Anlage nach einem der Ansprüche 2 bis 4 und Anspruch 5, dadurch gekennzeichnet, daß die erste Röntgenstrahlquelle (13) längsbeweglich in der Kippkonstruktion des Untersuchungstisches (11) montiert ist.

7. Anlage nach Anspruch 6, dadurch gekennzeichnet, daß die erste Quelle (13) an der unteren Verlängerung (19) der Säule montiert ist und sich mit dieser fortbewegt.

8. Anlage nach Anspruch 6, dadurch gekennzeichnet, daß die erste Quelle (13) an einem Zwischensupport befestigt ist, der verschiebbar in den quer zur unteren Verlängerung (19) verlaufenden Gleitschienen (21) gelagert ist.

FIG_1

0 147 334

# FIG_2

FIG_3

0 147 334